# EUROPEAN PATENT APPLICATION

(11) **EP 3 263 074 A1**
(43) Date of publication of application: **03.01.2018**
(21) Application number: 16755126.6
(22) Date of filing: 28.01.2016
(51) Int. Cl.: A61F 2/966, A61F 2/958

(54) **STENT DELIVERY DEVICE**

(30) Priority: 27.02.2015 JP 2015039353
(71) Applicant: Terumo Kabushiki Kaisha, Tokyo 151-0072 (JP)
(72) Inventor: KUMAZAWA, Takashi, Ashigarakami-gun Kanagawa 259-0151 (JP); TANI, Kazuyoshi, Ashigarakami-gun Kanagawa 259-0151 (JP)
(74) Representative: Casalonga
(86) International application number: PCT/JP2016/052544
(87) International publication number: WO 2016/136376

(57) **Abstract**

[Problem] In order to provide a stent delivery device capable of smoothly performing a procedure when a device is inserted into a living body.

[Means for Resolution] A stent delivery device 100 includes: a shaft 110 internally provided with lumens 113 and 114; a balloon 120 inflated and deflated around an axial direction of the shaft; a cover 130 configured to advance and retreat in the axial direction of the shaft between a covering position where the cover covers the deflated balloon and a retreated position where the cover is separated from the balloon; and a drive unit 150 configured to apply a driving force for advancing and retreating the cover.

The balloon and the drive unit are operated by a working fluid injected or discharged through the lumen of the shaft, and the inflation and deflation of the balloon and the advancing and retreating of the cover are performed in conjunction with each other.

## Description

### [Technical Field]

The present invention relates to a stent delivery device.

### [Background Art]

As treatment for a stenosed site or an occluded site generated in a vessel of a living body, the stenosed site or the occluded site is expanded by a stent to secure the vessel. The stent is delivered to a target site and is then expanded and detained therein. For example, in the technique discussed in Patent Document 1, after the stent is delivered to the target side, the stent is expanded by a balloon and is detained.

In a case where a coat layer such as a drug layer is formed on a surface of the stent, the stent and the inner wall of the vessel of the living body interfere with each other while the stent is delivered to the target site. This may damage the coat layer. Therefore, it is desirable to protect the stent. As an example of the stent protection means, the stent may be covered by a cover.

In this case, the stent is delivered to a target site of the living body while the stent is covered by the cover. Then, the stent is expanded along with the balloon. When the stent is expanded, the cover is moved to a position separated from the balloon and the stent in order to prevent inference. After the stent is detained, the balloon is deflated, and the cover is retracted to its original position to cover the balloon.

### [Citation List]

### [Patent Literature]

[PTL 1] Pamphlet of International Publication No. 2002/028319

### [Summary of Invention]

### [Technical Problem]

However, in order to move the cover separately from the operation for inflating or deflating the balloon, a procedure becomes complicated, so that it may difficult to effectively perform the procedure. If the procedure can be more effectively performed while the device is inserted into a living body, it is possible to reduce a patient's burden, which is particularly desirable.

The invention has been made in view of the aforementioned problems, and an object thereof is to provide a stent delivery device capable of performing a smooth procedure when a device is inserted into a living body.

### [Solution to Problem]

A stent delivery device of the invention for attaining the above-described object includes: a shaft internally provided with a lumen; a balloon inflated or deflated around an axial direction of the shaft; a cover configured to advance or retreat in the axial direction of the shaft between a covering position where the cover covers the deflated balloon and a retreated position where the cover is separated from the balloon; and a drive unit configured to apply a driving force for advancing or retreating the cover. The balloon and the drive unit are operated by a working fluid injected or discharged through the lumen of the shaft, and the inflation and deflation of the balloon and the advancing and retreating of the cover are performed in conjunction with each other.

### [Advantageous Effects of the Invention]

In the stent delivery device configured as described above, the cover is automatically moved by the drive unit such that the balloon is inflated or deflated by injecting or discharging the working fluid. For this reason, it is not necessary to separately perform an operation for inflating or deflating the balloon and an operation for moving the cover. In addition it is possible to smoothly perform a procedure when a device is inserted into a living body.

### [Brief Description of Drawings]

[Fig. 1] Fig. 1 is a diagram illustrating a stent delivery device according to a first embodiment.
[Fig. 2] Fig. 2 is a diagram illustrating the stent delivery device according to the first embodiment attached with a stent.
[Fig. 3] Fig. 3 is a diagram illustrating the stent delivery device according to the first embodiment when a sheath is apart from the balloon and moved to a retreated position.
[Fig. 4] Fig. 4 is a diagram illustrating the stent delivery device according to the first embodiment when the balloon is inflated along with the stent.
[Fig. 5] Fig. 5 is a diagram illustrating the stent delivery device according to the first embodiment when the sheath is moved to a covering position in which the sheath covers the balloon.
[Fig. 6] Fig. 6 is a diagram illustrating a stent delivery device according to the second embodiment.
[Fig. 7] Fig. 7 is a diagram illustrating a stent delivery device according to the third embodiment.
[Fig. 8] Fig. 8 is a diagram illustrating a modification of the sheath.

### [Description of Embodiments]

Embodiments according to the invention will now be described with reference to the accompanying drawings. Note that the dimensions or scales on the drawings may be exaggerated for convenient description purposes and are different from those of the reality.

### <First Embodiment>

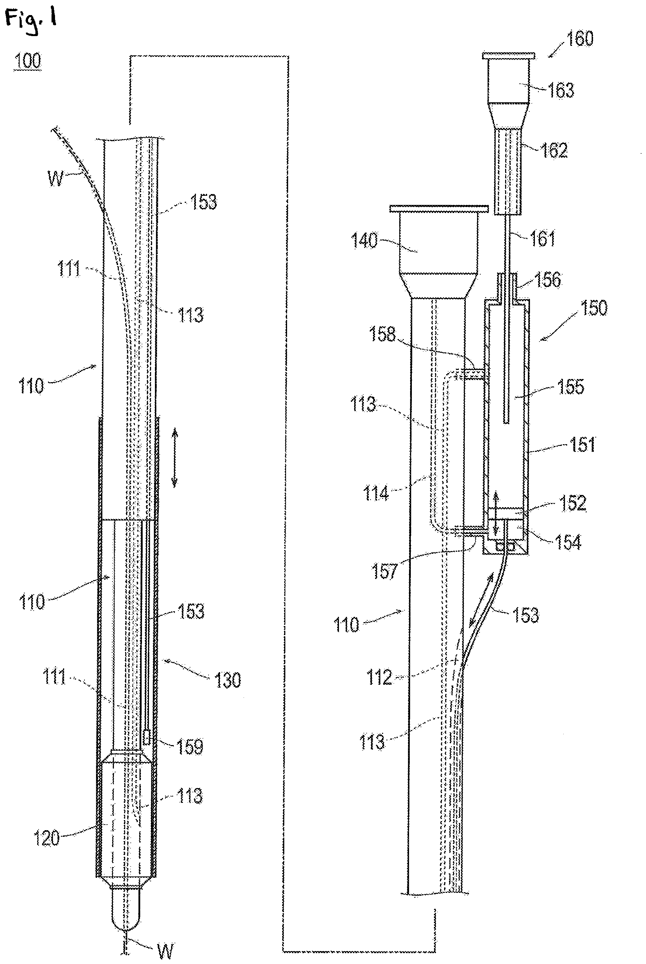

As illustrated in Fig. 1, a stent delivery device 100 according to the first embodiment includes a shaft 110, a balloon 120, a sheath 130 (cover), a hub 140, a drive unit 150, and a priming device 160.

The shaft 110 is provided with a guidewire lumen 111, an insertion hole 112, and lumens 113 and 114. The shaft 110 is flexible.

A guidewire W is inserted into the guidewire lumen 111. The guidewire lumen 111 extends in an axial direction from the most distal end of the shaft 110 to the proximal end side and is bent toward the outer circumferential surface of the shaft 110 in the proximal end side relative to the sheath 130. Without limiting to such a configuration, the guidewire lumen 111 may extend in the axial direction from the most distal end of the shaft 110 to the hub 140 and penetrate the shaft 110 and the hub 140 in the axial direction.

The insertion hole 112 communicates with the outside of the shaft 110 on the outer circumferential surface of the shaft 110 in the distal end side relative to the drive unit 150. In addition, although not illustrated in the drawings, the insertion hole 112 extends to the distal end side in the axial direction and communicates with the sheath 130.

The lumen 113 communicates with the inside of the balloon 120. The lumen 114 communicates with the hub 140. The lumens 113 and 114 do not directly communicate with each other but communicate through the drive unit 150.

The balloon 120 is provided in the distal end side of the shaft 110 and is bonded to an outer circumference of the shaft 110 in both ends of the axial direction. The balloon 120 has a hollowed cylindrical shape and can be inflated or deflated around the axial direction of the shaft 110.

The balloon 120 may be formed of a material having a relatively high elastic property and may be inflated such that the material expands as the internal pressure increases. Alternatively, the balloon 120 may be formed of a material having a relatively low elastic property so that it can be folded around the shaft 110 in a deflated state and can be unfolded and inflated as the internal pressure increases. The balloon 120 may be formed of a material similar to that of the balloon well known in the art to expand the stent.

The sheath 130 is provided so as to freely advance and retreat between a covering position in which the sheath 130 covers the deflated balloon 120 as illustrated in Fig. 1 and a retreated position in which the sheath 130 deviates from the covering position to the proximal end side in the axial direction and is separated from the balloon 120.

The surface of the sheath 130 is preferably formed of a material having a low frictional resistance. Using such a material, the sheath 130 can smoothly advance or retreat with respect to the balloon 120 and the shaft 110 while friction therewith is suppressed. A material of the surface of the sheath 130 is, for example, polytetrafluoroethylene (PTFE). The sheath 130 may be radiopaque.

The hub 140 is provided in the proximal end of the shaft 110 and is connected to a supply/discharge device such as an indeflator (not illustrated) that supplies or discharges the working fluid. The working fluid includes, for example, a contrast medium or a mixture of the contrast medium and physiological saline, but not limited thereto.

The drive unit 150 has a cylinder 151 (housing portion) and a piston 152. In addition, the drive unit 150 has a wire 153 as a driving force transmission portion that transmits a force to the sheath 130.

The piston 152 is slidably housed in the cylinder 151 and slides along the axial direction. The cylinder 151 is formed such that a motion and a position of the piston 152 of the axial direction can be visually recognized from the outside of the cylinder 151. The inside of the cylinder 151 is divided into a first chamber 154 and a second chamber 155 by the piston 152.

The cylinder 151 has a ventilation hole 156 that allows the second chamber 155 to communicate with the outside. The ventilation hole 156 is provided in the proximal end of the cylinder 151. As the piston 152 slides along the axial direction, the external air flows to the second chamber 155 through the ventilation hole 156, or the air is discharged to the outside from the second chamber 155.

The cylinder 151 has a first communication portion 157 that communicates with the first chamber 154. The cylinder 151 has a second communication portion 158 that communicates with the second chamber 155. Each of the first and second communication portions 157 and 158 protrudes perpendicularly to the axial direction.

A fitting hole that allows insertion and removal of each of the first and second communication portions 157 and 158 is provided on the outer circumferential surface of the proximal end side of the shaft 110. By inserting or removing the first and second communication portions 157 and 158 to the fitting hole, the drive unit 150 is detachably attached to the shaft 110.

If the drive unit 150 is attached to shaft 110, the first communication portion 157 communicates with the lumen 114, and the second communication portion 158 communicates with the lumen 113. As a result, the working fluid is injected into or discharged from the drive unit 150, so that the drive unit 150 has an operable state. Meanwhile, when the drive unit 150 is removed from the shaft 110, the working fluid is not injected into or discharged from the drive unit 150. Therefore, the drive unit 150 has an inoperable state.

The wire 153 connects the piston 152 and the sheath 130. The wire 153 is slidably extracted from the cylinder 151. The wire 153 is extended to the inside of the sheath 130 through the insertion hole 112. The wire 153 is bonded to the inner circumferential surface of the sheath 130 in a bonding portion 159 provided in the distal end.

A material of the wire 153 may include, for example, metal or resin. The wire preferably has a certain degree of stiffness in order to transmit an axial force applied to the piston 152 to the sheath 130, but not particularly limited thereto.

The priming device 160 includes a piston rod 161, a priming tube 162, and a hub 163.

The piston rod 161 is a bar-like member and protrudes from an opening of the distal end of the priming tube 162. The piston rod 161 is inserted into the inside of the cylinder 151 from the ventilation hole 156.

The priming tube 162 is detachably attached to the ventilation hole 156. The priming tube 162 is connected to the ventilation hole 156 to communicate with the second chamber 155.

The hub 163 has a hollow shape and communicates with the priming tube 162. The proximal end of the hub 163 is connected to a supply/discharge device such as a syringe (not illustrated) that supplies or discharges the priming liquid. The priming liquid includes, for example, physiological saline, a contrast medium, or a mixture of them.

Next, the operation of the stent delivery device 100 will be described.

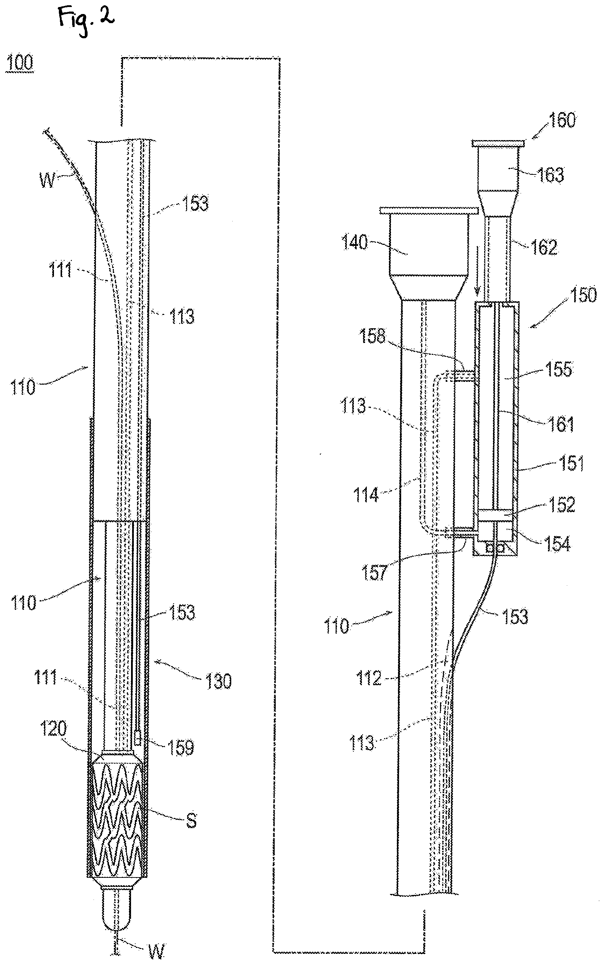

As illustrated in Fig. 2, the stent delivery device 100 delivers the stent S to a lesion such as a stenosed site or an occluded site generated in a vessel of the living body while the stent S attached to the outer circumference of the deflated balloon 120 is covered by the sheath 130. The distal end side of the stent delivery device 100 is inserted into a vessel, and the hub 140, the drive unit 150, and the priming device 160 are used outside the living body. The vessels to which the stent delivery device 100 is applied may include, for example, a blood vessel, a bile duct, an esophagus, a trachea, a urethra, or the like, but not particularly limited thereto.

The stent S has a mesh tubular shape, and its shape and material are not particularly limited. The stent S is formed of, for example, metal or resin. Alternatively, a part of the stent S may be formed of resin, and the other parts may be formed of metal. The stent S may have biodegradability. In addition, the stent S may have a coat layer such as a drug layer on its surface.

The stent delivery device 100 is delivered to a target lesion along the guidewire W introduced into a vessel in advance. As the guidewire W is inserted into the guidewire lumen 111, the distal end side of the stent delivery device 100 is moved along the guidewire W without being apart from the guidewire W. After the distal end side of the stent delivery device 100 reaches a target lesion, the stent S is detained.

Prior to detaining of the stent S, priming is performed such that the air in the balloon 120, the shaft 110, and the cylinder 151 is discharged, and the priming liquid is filled therein.

For the priming, the priming device 160 is attached to the cylinder 151. In this case, the piston rod 161 abuts on the piston 152, and the priming tube 162 communicates with the cylinder 151. In this state, the supply/discharge device (not illustrated) is connected to the proximal end of the hub 163.

As the supply/discharge device connected to the hub 163 suctions the air, the air is discharged from the balloon 120, the lumen 113, and the second chamber 155 through the priming tube 162 and the hub 163. In this case, the piston 152 abuts on the piston rod 161, so that sliding toward the proximal end side is restricted. The piston rod 161 restricts the piston 152 from moving toward the proximal end side over the second communication portion 158, so that the piston 152 does not hinder the air from being discharged from the lumen 113 and the balloon 120 through the second communication portion 158.

The supply/discharge device connected to the hub 163 supplies the priming liquid after the air is discharged. The priming liquid flows from the priming tube 162 to the second chamber 155 and is filled in the lumen 113 and the balloon 120 through the second communication portion 158.

By filling the priming liquid in the lumen 113 and the balloon 120, it is possible to make it easy to apply a pressure to the inside of the balloon 120 to inflate the balloon 120. In addition, since the air is discharged by performing priming, it is possible to reduce a possibility that the air contained in the stent delivery device 100 is input to the living body.

After the priming, the priming device 160 is removed from the cylinder 151 to extract the piston rod 161. As a result, the piston rod 161 is separated from the piston 152, and the restriction is released. Therefore, the piston 152 can freely slide inside the cylinder 151. Then, the stent S is detained in the vessel.

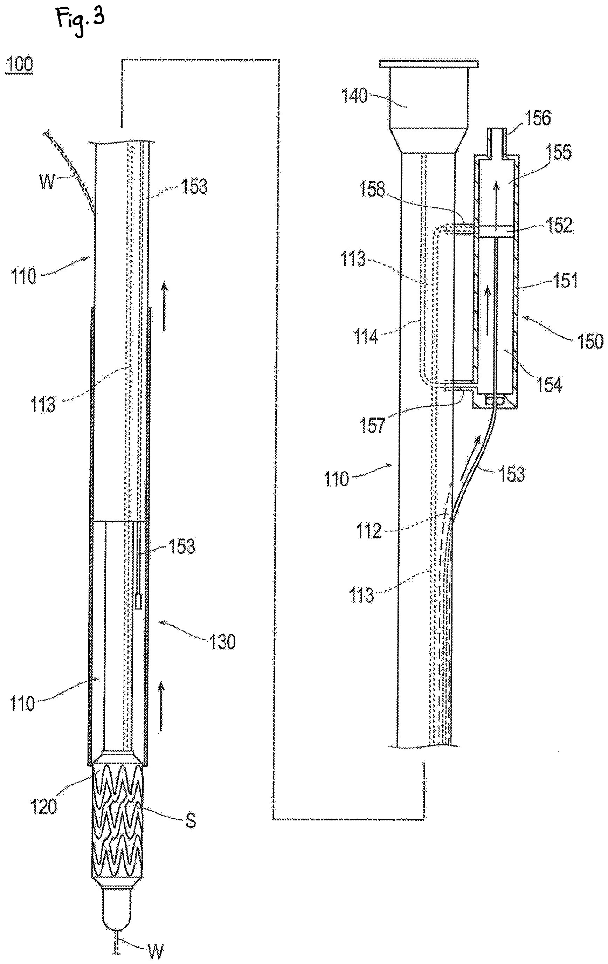

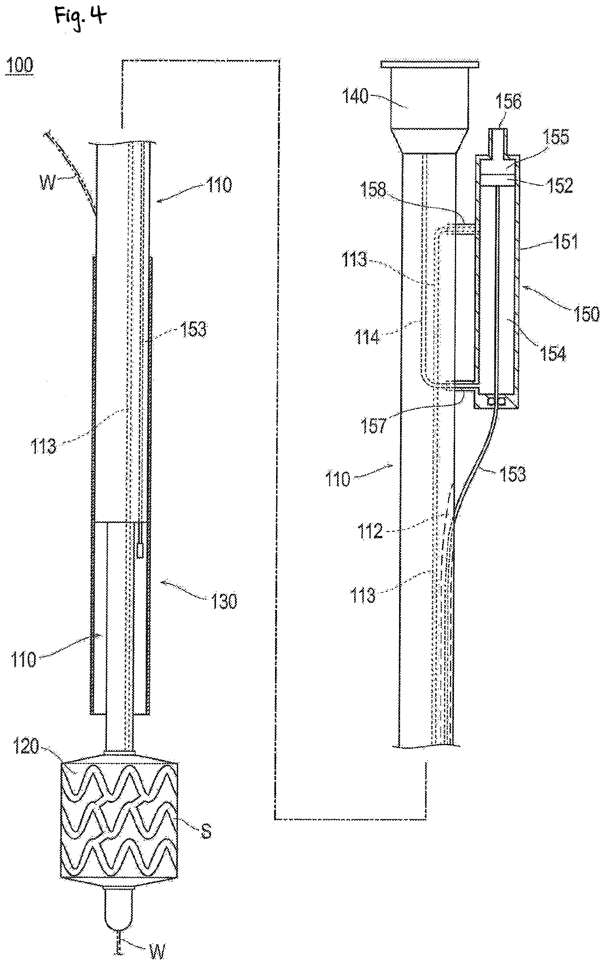

In order to detain the stent S, the drive unit 150 moves the sheath 130 toward the proximal end side in the axial direction so that the sheath 130 is apart from the balloon 120 and the stent S as illustrated in Fig. 3, so that the balloon 120 inflates the stent S as illustrated in Fig. 4. The drive unit 150 and the balloon 120 are operated by the working fluid introduced into the shaft 110.

Prior to the start of inflation of the balloon 120, the drive unit 150 retreats the sheath 130 to a retreated position apart from the balloon 120 and the stent S. Then, the balloon 120 is inflated together with the stent S. The sheath 130 is moved and the balloon 120 is inflated in conjunction with introduction of the working fluid into the shaft 110.

The working fluid is supplied from a supply/discharge device connected to the hub 140 and is introduced into the first chamber 154 through the lumen 114 and the first communication portion 157. As the working fluid is introduced into the first chamber 154, the piston 152 receives a force applied to the proximal end side in the axial direction. This force (driving force) is transmitted to the sheath 130 through the wire 153. As a result, the sheath 130 is moved toward the proximal end side in the axial direction.

While the piston 152 is placed in the distal end side relative to the second communication portion 158, the first chamber 154 does not communicate with the second communication portion 158 and the lumen 113 as illustrated in Fig. 3, and the working fluid is not supplied to the balloon 120. Therefore, the balloon 120 is not inflated. Meanwhile, the sheath 130 is pulled toward the proximal end side in the axial direction along with the move of the piston 152 and is moved to the retreated position apart from the balloon 120. When the sheath 130 is moved to the retreated position, both the balloon 120 and the stent S are exposed to the outside of the sheath 130.

As the piston 152 is moved to the proximal end side over the second communication portion 158, the first chamber 154 communicates with the second communication portion 158 and the lumen 113 as illustrated in Fig. 4. Therefore, the working fluid is supplied to the balloon 120 so as to inflate the balloon 120. In this case, since the sheath 130 is placed in the retreated position, it does not hinder inflation of the balloon 120 and the stent S.

The stenosed site or the occluded site in the vessel is inflated as the balloon 120 is inflated. Then, the stent delivery device 100 detains the stent S and is removed from the vessel.

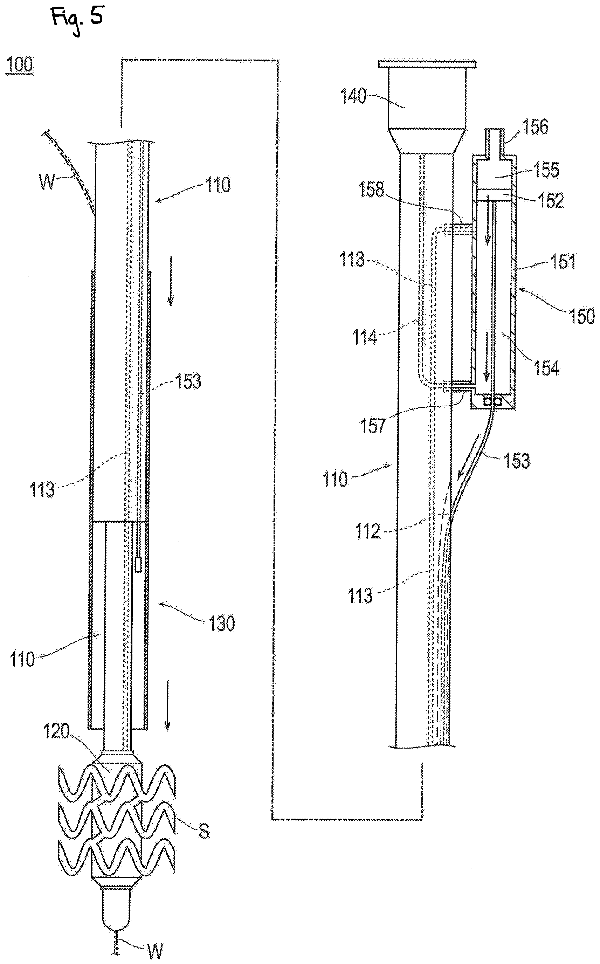

In this case, as illustrated in Fig. 5, the stent delivery device 100 deflates the balloon 120, and the sheath 130 covers the deflated balloon 120. The expanded stent S is maintained in the expansion state even when the balloon 120 is deflated. The stent S is detained in the vessel inside the living body in the expanded state and holds the stenosed site or the occluded site of the vessel in the expansion state.

The balloon 120 is deflated by discharging the working fluid through the lumen 113. The working fluid discharged from the balloon 120 is suctioned to the supply/discharge device connected to the hub 140 through the lumen 113, the second communication portion 158, the first chamber 154, the first communication portion 157, and the lumen 114.

This suctioning causes the working fluid to be discharged from the first chamber 154 through the first communication portion 157. As a result, the piston 152 is moved to the distal end side in the axial direction, and the drive unit 150 transmits a force (driving force) applied toward the distal end side of the piston 152 in the axial direction to the sheath 130 through the wire 153. For this reason, the sheath 130 is moved to the covering position in conjunction with the deflation of the balloon 120.

Here, since the balloon 120 is relatively easily deflated after the stent S is removed, the deflation of the balloon 120 starts before the move of the piston 152 and the sheath 130. Therefore, the sheath 130 starts to be moved after the balloon 120 is deflated.

The sheath 130 is moved to the covering position in which the sheath 130 covers the entire deflated balloon 120 (refer to Fig. 1). Then, the stent delivery device 100 is removed from the vessel.

Next, functional effects of this embodiment will be described.

Unlike this embodiment, for example, in a case where an operator moves the sheath 130 by manually pulling the wire 153, the sheath 130 is moved to the retreated portion before the working fluid is introduced into the shaft 110 to inflate the balloon 120. Therefore, the operator is required to separately perform an operation for manually pulling the wire 153.

In this case, the deflated balloon 120 is covered by the sheath 130 again. Therefore, after the operation of deflating the balloon 120 by suctioning the working fluid from the balloon 120, an operator is required to move the sheath 130 to the covering position by manually pushing the wire 153 into the distal end side.

In contrast, in the stent delivery device 100 according to this embodiment, the sheath 130 is automatically moved by the drive unit 150 as the balloon 120 is inflated or deflated by injecting or discharging the working fluid to or from the shaft 110. For this reason, it is not necessary to perform a separate operation for inflating and deflating the balloon 120 and moving the sheath 130 unlike the aforementioned example. Therefore, it is possible to effectively inflate and deflate the balloon 120 and move the sheath 130 and smoothly perform a procedure while the stent delivery device 100 is inserted into the vessel.

The cylinder 151 is formed to be transparent so that the piston 152 is visual from the outside. For this reason, even when the distal end side of the stent delivery device 100 is inserted into the body, an operator can easily and simply predict a position or motion of the sheath 130 which is moved along with the piston 152 by visually checking a position or motion of the piston 152. In addition, an operator can accurately adjust a position or motion of the sheath 130 on the basis thereof.

For the priming, the piston 152 abuts on the piston rod 161 so that its movement is restricted. For this reason, the sheath 130 connected to the piston 152 does not move, and it is possible to prevent the sheath 130 from moving by the priming unintentionally. More specifically, when the air of the second chamber 155 is suctioned by the priming, the piston rod 161 abuts on the piston 152, and the move of the piston 152 toward the proximal end side is restricted. Therefore, it is possible to prevent the sheath 130 from being unintentionally moved to the retreated position during the priming.

The drive unit 150 moves the sheath 130 to the retreated position before a start of the inflation of the balloon 120. For this reason, the move of the sheath 130 toward the retreated position is not interrupted by the inflation of the balloon 120. In addition, the inflation of the balloon 120 is not interrupted by the sheath 130. Therefore, it is possible to reliably move the sheath 130 toward the retreated position and inflate the balloon 120.

The drive unit 150 moves the sheath 130 to the covering position after a start of the deflation of the balloon 120. For this reason, when the sheath 130 reaches the covering position, the balloon 120 is already deflated. Therefore, the sheath 130 and the balloon 120 do not easily interfere with each other, and the balloon 120 can be easily fitted into the sheath 130.

The drive unit 150 is not operated while it is removed from the shaft 110. Therefore, by removing the drive unit 150 from the shaft 110, it is possible to prevent the sheath 130 from unintentionally advancing or retreating and prevent the balloon 120 from being unintentionally inflated or deflated. Accordingly, it is possible to prevent an erroneous operation.

### <Second Embodiment>

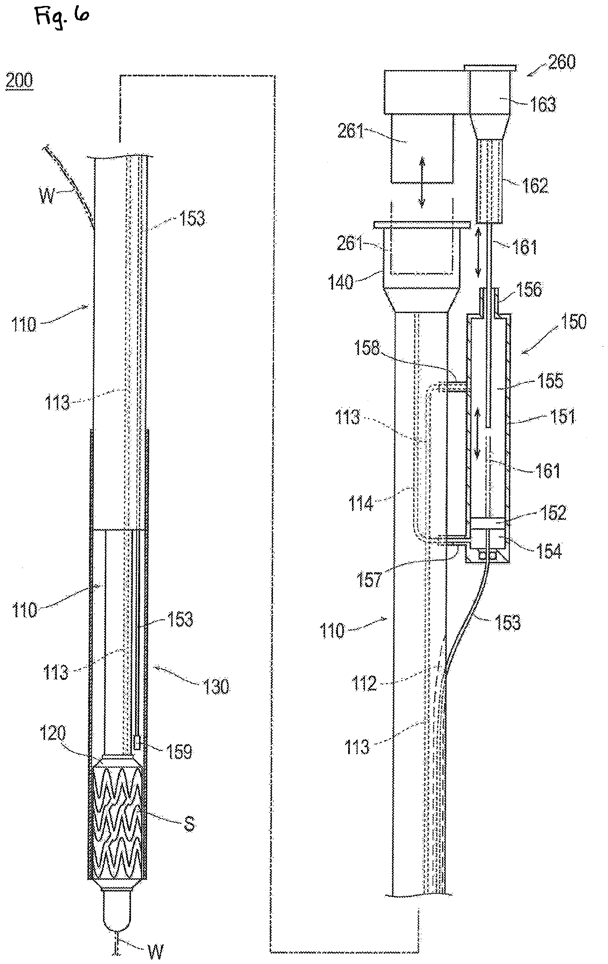

A stent delivery device 200 according to the second embodiment illustrated in Fig. 6 has a priming device 260 different from that of the first embodiment. Other configurations of the stent delivery device 200 are similar to those of the stent delivery device 100 of the first embodiment, and they will not be described repeatedly by allocating the same reference signs and numerals.

The priming device 260 has a piston rod 161, a priming tube 162, a hub 163, and a hub 261 (plug member). The hub 261 is formed integrally with the piston rod 161, the priming tube 162, and the hub 163. The piston rod 161, the priming tube 162, and the hub 163 are similar to those of the first embodiment.

1 As the piston rod 161 is inserted into the cylinder 151, the hub 261 is inserted into the hub 140 to block the hub 140. As a result, the priming liquid is not erroneously injected into the hub 140. Therefore, it is possible to prevent an erroneous operation of the piston 152 and prevent the sheath 130 from being unintentionally moved during the priming. More specifically, since the priming liquid is not erroneously injected into the first chamber 154 through the hub 140 and the lumen 114, it is possible to prevent the sheath 130 from being unintentionally moved to the retreated position during the priming.

In addition, since the hub 261 is integrally assembled with the piston rod 161, the hub 140 is reliably blocked as the piston rod 161 is inserted into the cylinder 151. Therefore, it is possible to prevent an operator from forgetting to block the hub 140.

Other functional effects according to this embodiment caused by components common to the first embodiment are similar to those of the first embodiment.

After the priming, the hub 261 is extracted along with the piston rod 161 and is removed from the hub 140.

### <Third Embodiment>

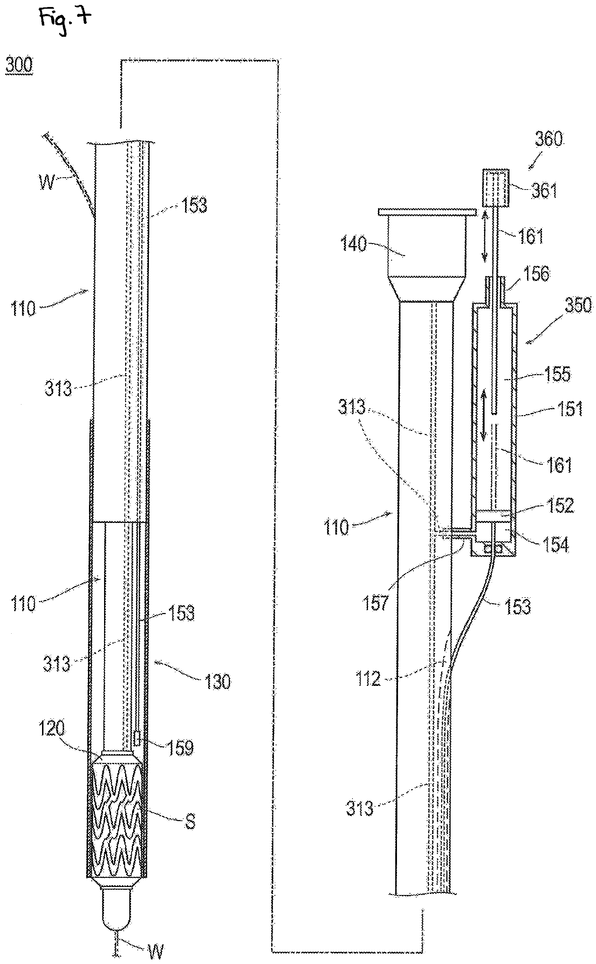

A stent delivery device 300 according to a third embodiment illustrated in Fig. 7 has a drive unit 350, a lumen 313, and a priming device 360 different from those of the first embodiment. Other configurations of the stent delivery device 300 are similar to those of the first embodiment, and they will not be described repeatedly by allocating the same reference signs and numerals.

The lumen 313 communicates with the balloon 120 in the distal end side. The lumen 313 is branched in the proximal end side so as to communicate with the drive unit 350 and the hub 140. The drive unit 350 according to the third embodiment is different from the drive unit 150 of the first embodiment in that the second communication portion 158 is removed.

The priming device 360 has a piston rod 161 and a hub 361. The piston rod 161 is similar to that of the first embodiment. The hub 361 is detachably attached to the ventilation hole 156.

For the priming, the hub 361 is connected to the ventilation hole 156 to block the ventilation hole 156. In this case, the piston rod 161 abuts on the piston 152 to restrict the piston 152 from sliding toward the proximal end side. According to this embodiment, the priming is performed using a supply/discharge device (not illustrated) connected to the hub 140.

As the supply/discharge device connected to the hub 140 suctions the air, the air from the lumen 313 and the balloon 120 is discharged. In this case, since the hub 361 blocks the ventilation hole 156, the air does not flow to the cylinder 151.

The supply/discharge device connected to the hub 140 supplies the priming liquid after the air is discharged. The priming liquid is filled in the lumen 313 and the balloon 120 through the hub 140. In this case, since the piston rod 161 abuts on the piston 152 to restrict sliding toward the proximal end side, it is possible to prevent an unintentional movement of the sheath 130 connected to the piston 152.

After the priming, the priming device 360 is removed from the cylinder 151 by extracting the piston rod 161, and the piston rod 161 is separated from the piston 152. As a result, the restriction to the piston 152 is released, and the piston 152 can freely slide inside the cylinder 151.

Then, the working fluid is supplied from the supply/discharge device connected to the hub 140 to the shaft 110 to move the sheath 130 and inflate the balloon 120 and the stent S.

The working fluid flows to the first chamber 154 through the lumen 313 and the communication portion 157. As a result, the piston 152 is moved toward the proximal end side in the axial direction, and the sheath 130 is pulled toward the proximal end side in the axial direction using the wire 153 and is moved to the retreated position.

In this case, the working fluid also flows to the balloon 120 through the lumen 313. However, since an inflation pressure P2 necessary to inflate the balloon 120 attached with the stent S is higher than a pressure P1 necessary to slide the piston 152 (P2 > P1), the balloon 120 is not inflated or is very slightly inflated.

The pressure P1 necessary to slide the piston 152 is set to, for example, 1.5 to 2 atm. The inflation pressure P2 for the balloon 120 attached with the stent S is set to, for example, 3 to 4 atm.

After the sheath 130 is moved to the retreated position, an operator increases the pressure of the working fluid supplied to the shaft 110. When the pressure is equal to or higher than the inflation pressure P2, the balloon 120 is inflated along with the stent S.

After the stent S is detained, the working fluid is discharged to the supply/discharge device connected to the hub 140 through the lumen 313.

An absolute value P3 of the negative pressure necessary to deflate the balloon 120 after removing the stent S is smaller than the absolute value P1 of the negative pressure necessary to slide the piston 152 (P1 > P3). For this reason, as the supply/discharge device connected to the hub 140 suctions the working fluid, the balloon 120 is deflated before the piston 152 starts to be moved. Then, when the balloon 120 is further depressurized, the piston 152 is moved toward the distal end side in the axial direction. As a result, the sheath 130 is moved to the covering position to cover the deflated balloon 120.

Since the stent delivery device 300 has the same function as that of the first embodiment, the same effects as those of the first embodiment can be obtained from the third embodiment. Note that, according to the third embodiment, the piston rod 161 may not be necessary by optimizing a sliding resistance so as not to excessively lower friction between the piston 152 and the cylinder 151. As a result, according to the third embodiment, it is possible to reduce cumbersomeness or cost by simplifying the entire system.

The present invention is not limited to the aforementioned embodiments, and various modifications may be possible without departing from the scope of the claims.

For example, the drive unit may be fixed to the shaft instead of being detachably attached. In addition, a housing portion that houses the piston so that the piston is slidable may be provided in the shaft itself.

In the drive units 150 and 350 according to the aforementioned embodiments, it is not necessary to form the entire cylinder 151 with a transparent material. A window through with the piston 152 is visible may be provided in a part of the cylinder 151.

In the aforementioned embodiment, the drive units 150 and 350 start the move of the sheath 130 at the timing deviated from the inflation/deflation timing of the balloon 120. However, the present invention is not limited thereto. Alternatively, the inflation/deflation of the balloon and the move of the sheath may start simultaneously. For example, even when the deflation of the balloon and the move of the sheath to the covering position start simultaneously, the sheath and the balloon do not interfere with each other, and the balloon can be easily inserted into the sheath if the balloon is deflated when the sheath reaches the balloon.

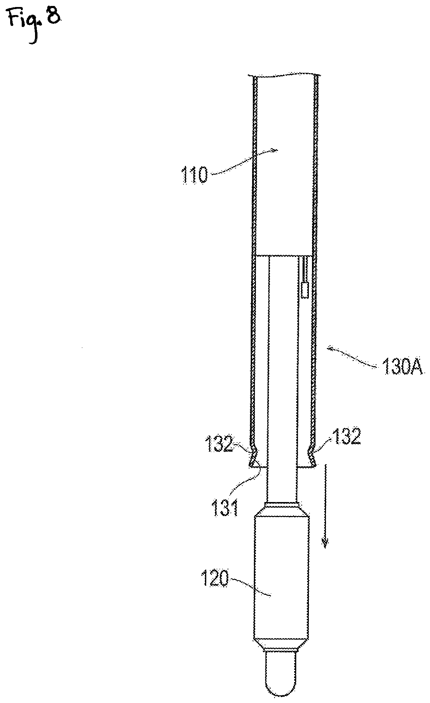

In addition, the cover according to the invention is not limited to the sheath 130 of the aforementioned embodiments. The cover according to the present invention may include, for example, a sheath 130A illustrated in Fig. 8.

The sheath 130A is shaped to have a tapered portion 131 in the distal end in the retreated position. The tapered portion 131 is opened to be widened toward the distal end side in the axial direction. A protrusion 132 protruding inward in the radial direction is formed in the proximal end side of the tapered portion 131.

The sheath 130A is formed of a flexible material. Therefore, when the sheath 130A is moved to the covering position to cover the balloon 120, the protrusion 132 is deformed to follow the shape of the balloon 120 and has a tubular shape such as the sheath 130 of Fig. 1.

Since the sheath 130A has the tapered portion 131 in the distal end in the retreated position, the balloon 120 is smoothly guided to the sheath 130A using the tapered portion 131 when the sheath 130A is moved to the covering position to cover the balloon 120. Therefore, it is possible to easily insert the balloon 120 into the sheath 130A.

This application is based upon and claims the benefit of priority from Japanese Patent Application No. 2015-039353 filed on February 27, 2015, the entire contents of which are incorporated herein by reference.

### [Reference Signs List]

- 100, 200, 300: stent delivery device,
- 110: shaft,
- 111: guidewire lumen,
- 112: insertion hole,
- 113, 114, 313: lumen,
- 120: balloon,
- 130, 130A: sheath (cover),
- 131: tapered portion,
- 132: protrusion,
- 140: hub,
- 150, 350: drive unit,
- 151: cylinder (housing portion),
- 152: piston,
- 153: wire,
- 154: first chamber,
- 155: second chamber,
- 156: ventilation hole,
- 157: first communication portion,
- 158: second communication portion,
- 159: bonding portion,
- 160, 260, 360: priming device,
- 161: piston rod,
- 162: priming tube,
- 163: hub,
- 261: hub (plug member),
- 361: hub,
- S: stent,
- W: guidewire.

## Claims

1. A stent delivery device comprising:
a shaft internally provided with a lumen;
a balloon inflated or deflated around an axial direction of the shaft;
a cover configured to advance or retreat in the axial direction of the shaft between a covering position where the cover covers the deflated balloon and a retreated position where the cover is separated from the balloon; and
a drive unit configured to apply a driving force for advancing or retreating the cover,
wherein the balloon and the drive unit are operated by a working fluid injected or discharged through the lumen of the shaft, and
the inflation and deflation of the balloon and the advancing and retreating of the cover are performed in conjunction with each other.

2. The stent delivery device according to claim 1, wherein the drive unit has a piston connected to the cover and a housing portion configured to house the piston so that the piston is slidable, and
at least a part of the housing portion is formed so that the piston is visual.

3. The stent delivery device according to claim 1 or 2, wherein the drive unit has a piston connected to the cover and a housing portion configured to house the piston so that the piston is slidable,
the housing portion is provided with a piston rod that abuts on and retreats from the piston to restrict the piston from sliding and release the restriction.

4. The stent delivery device according to claim 3, wherein the piston rod is inserted into the housing portion from a proximal end of the housing portion, and
a plug member formed integrally with the piston rod blocks a hub communicating with the lumen in the proximal end of the shaft as the piston rod is inserted.

5. The stent delivery device according to any one of claims 1 to 4, wherein the drive unit performs at least one of move of the cover toward the retreated position before starting the inflation of the balloon or move of the cover toward the covering position after starting the deflation of the balloon.

6. The stent delivery device according to any one of claims 1 to 5, wherein the drive unit is detachably attached to the shaft,
the drive unit is not operated when the drive unit is detached from the shaft, and
the drive unit is operable when the drive unit is attached to the shaft.

7. The stent delivery device according to any one of claims 1 to 6, wherein a distal end portion of the cover is shaped to have a tapered portion opened to be widened toward a distal end side of the axial direction in the retreated position.

8. The stent delivery device according to any one of claims 1 to 7, wherein a stent is attached to an outer circumference of the balloon, and
the stent is covered by the cover.
